# EUROPÄISCHE PATENTSCHRIFT

(11) **EP 0 675 176 B1**
(45) Veröffentlichungstag und Bekanntmachung des Hinweises auf die Patenterteilung: **28.07.1999**
(21) Anmeldenummer: 95103549.2
(22) Anmeldetag: 11.03.1995
(51) Int. Cl.: C09D 17/00, C09C 1/42, C01B 33/44

(54) **Verdickungsmittel auf der Basis mindestens eines synthetischen Schichtsilicats**
Thickening agent comprising at least one synthetic layered silicate
Agent épaississant à base d'au moins un silicate synthétique en couches

(30) Priorität: 28.03.1994 DE 4410727
(43) Veröffentlichungstag der Anmeldung: 04.10.1995
(73) Patentinhaber: SÜD-CHEMIE AG, 80333 München (DE)
(72) Erfinder: Coutelle, Helmut, Dr., D-85356 Freising (DE); Heininger, Wolfgang, Dr., D-85368 Moosburg (DE); Buckl, Wolfgang, D-85413 Hörgertshausen (DE)
(74) Vertreter: Patentanwälte Dipl.-Ing. R. Splanemann Dr. B. Reitzner Dipl.-Ing. K. Baronetzky

(56) Entgegenhaltungen:
- DE-A- 4 404 218
- GB-A- 2 164 950
- GB-A- 2 193 724

## Beschreibung

Die Erfindung betrifft Verdickungsmittel auf der Basis mindestens eines synthetischen Schichtsilicats.

In flüssigen oder fließfähigen Beschichtungssystemen, wie Farben, Lacken, Klebstoffen, Druckfarben, Unterbodenschutzmassen, Bohrspülungen, kosmetischen und pharmazeutischen Rezepturen, werden Schichtsilicate eingesetzt, um eine Reihe von Problemen zu lösen, wie das Absetzen von schweren Pigmenten oder Wirkstoffen bei der Lagerung, die Bereitstellung der notwendigen Schichtdicke aus Schutzgründen, die Bereitstellung der für die Anwendung geeigneten Viskosität usw..

Diese Schichtsilicate können thixotrope Kartenhausstrukturen ausbilden, die die gewünschten Eigenschaften in das System bringen. Da diese Produkte selbst stark verdickend wirken, was ja der Grund für ihren Einsatz ist, werden die Materialien nicht in vordispergierter Form angeboten und in den Handel gebracht. Der wesentliche Grund dafür ist die Tatsache, daß nur 2 bis 8%ige Pasten als Suspension herstellbar und handhabbar sind. Damit würde im wesentlichen Wasser transportiert, was ökonomisch nicht sinnvoll ist.

Außerdem kann zuviel Wasser in eine Formulierung eingeschleppt werden, was unter anderem dazu führen kann, daß der gewünschte Verdickungseffekt des Schichtsilicats durch die hohe zusätzliche Wassermenge wieder zunichte gemacht wird. Der Verdünnungseffekt kann dann die Wirkung wieder aufheben.

Die gute Dispergierung von Pulvern ist jedoch bei allen Schichtsilicaten ein Problem. Entweder ist die Dispergierung nicht vollständig, und das Material wird nur teilweise ausgenutzt (was zudem auch zu anderen Problemen führt, wie Stippenbildung, Nachverdickungserscheinungen, Glanz- und Effizienzverlust), oder aber sie ist aufgrund fehlender Wasseranteile und damit der Möglichkeit, die fehlenden mechanischen Dispergierkräfte durch eine Anquellung mit Wasser auszugleichen, in der Formulierung nicht möglich. Selbst wenn die erforderliche mechanische Dispergierenergie zur Verfügung steht, kann in vielen Fällen die Dispergierung nicht vollständig durchgeführt werden, da die Rezepturbestandteile nicht immer scherstabil sind. Unkontrollierbare Nachverdickungseffekte sind daher die Folge; auch Stippenbildung, Agglomerate und Glanzverluste treten auf.

Die Zugabe von Dispergiermitteln zur Verbesserung der Dispergierung ist zwar ebenfalls Stand der Technik; diese Zugabe erfolgt aber im Normalfall erst, nachdem das Schichtsilicat in Wasser vorbenetzt ist. Der Grund hierfür ist die Tatsache, daß die meisten Dispergierhilfsmittel hochpolar sind uns sich so an die Kanten der Schichtsilicatlamellen anlagern können, daß die Hydratisierung sogar unterdrückt und der gegenteilige Effekt erreicht wird. Dispergierhilfsmittel, z.B. auf Acrylatbasis oder Acrylamidbasis, haben sogar die Tendenz, Suspensionen zu flocken, was natürlich die Viskosität eines Schichtsilicat-Gels senkt, aber am eigentlichen Ziel der guten Delaminierung und Dispergierung vorbeigeht.

Aus der US-A-4 382 868 ist ein Verfahren zur Herstellung von organophilen Tonen mit verbesserter Dispergierbarkeit in organischen Flüssigkeiten bekannt, wobei ein Gemisch aus einem smektitischen Ton, einer quaternären Ammonium- oder Phosphoniumverbindung, Wasser und einem Alkohol mit 1 bis 5 Kohlenstoffatomen extrudiert und das erhaltene Organoton-Gelmittel ohne Entfernung des Wassers oder Alkohols vermahlen wird. Bei dem Produkt handelt es sich nicht mehr um einen in Wasser quellbaren Ton.

Aus der US-A-5 128 067 ist ein flammhemmendes Schmiermittel bekannt, welches ein Gemisch aus Alkoxy und Phenyl-Triestern einer Phosphor-Oxysäure und ein feinteiliges kieselsäurehaltiges Verdickungsmittel, z.B. Ton und Kieselsäure, enthält. Es handelt sich hierbei aber nicht um ein Umsetzungsprodukt.

Aus der US-A-5 336 647 sind organophile Tone bekannt, die das Reaktionsprodukt eines smektitischen Tons eines ersten organischen Kations und eines zweiten organischen Kations aus einem polyalkoxilierten quaternären Ammoniumsalz darstellen. Der Ton liegt nicht mehr in hydrophiler Form vor.

Aus der DE-C-3 145 452 ist ein gelbildender organophiler Ton bekannt, der das Reaktionsprodukt eines Tons vom Smektit-Typ mit einem organischen Anion (z.B. aus einem Phosphit oder Phosphat) und einem organischen Kation, z.B. aus einer quaternären Ammonium- oder Phosphoniumverbindung, darstellt. Das Umsetzungsprodukt ist auch hier nicht mehr hydrophil.

Aus der DE-A-3 914 916 ist ein Mittel zum Viskositätsaufbau in nichtwäßrigen Flüssigphasen bekannt, das aus feindispersen Teilchen einer anorganischen Schichtverbindung mit positiver Schichtladung auf Basis zweidimensionaler anorganischer Polykationen (polykationische Schichtverbindungen) und ein- und/oder mehrbasische Säureanionen ausgeprägt oleophilen Charakters gebildet ist. Auch dieses Mittel ist nicht in Wasser quellbar.

Aus der DE-A-3 145 475 ist ein gelbildender organophiler Ton bekannt, der das Reaktionsprodukt eines organischen Kations, eines organischen Anions und eines Tons von Smektit-Typ darstellt, wobei das organische Kation aus der Gruppe der quaternären Ammoniumsalze, Phosphoniumsalze und/oder Sulfoniumsalze ausgewählt ist. Auch dieses Produkt ist nicht in Wasser quellbar.

Aus der DE-A-3 930 687 ist die Verwendung von Phosphorsäure-estern und deren Salzen als Dispergiermittel und Dispersionsstabilisatoren für Pigmente in Farben, Lacken und Kunstharzen bekannt. Als Pigmente sind Aluminiumhydroxid, Zinkstearat, Calciumcarbonat, Magnesmiumoxid und Ruß genannt.

Aus der DE-C-3 230 476 sind leicht dispergierbare Organosmektite bekannt, deren austauschbare Kationen zu 50% durch organische Kationen ersetzt sind und deren Oberfläche mit einem Peptisierungsmittel belegt ist, das aus der Gruppe Natriumtetrapolyphosphat, Natriumtripolyphosphat, Natriumsilicat, Pyrogallol, Gallussäure und Tannat ausgewählt ist. Auch dieses Produkt ist nicht mehr in Wasser quellbar.

Aus der Literaturstelle Kittel, Hans: Lehrbuch der Lacke und Beschichtungen, Verlag W.A.Colomb in der H.Heenemann GmbH, Berlin-Oberschwandorf, 1974, Bd. II, S. 340, 341, ist bekannt, daß Silicate, z.B. Mineralien vom Typ Montmorillonit, als Additive für Lacke verwendet werden können. Über eine Modifizierung dieser Substanzen zwecks besserer Dispergierbarkeit finden sich keine Angaben.

Aus der Literaturstelle Van Doren, Robert E.; et al.: The Role of Rheological Additives in Protective Coatings. In: Jornal of Protective Coatings & Linings, May 1989, S. 47-52, sind rheologische Zusätze oder Verdickungsmittel für pigmenthaltige Schutzüberzüge bekannt, wobei u.a. pyrogene Kieselsäuren, celluloshaltige Verdickungsmittel, Acrylate und Polyether-Polyurethane genannt sind.

Die Aufgabe der Erfindung war daher, die Dispergierbarkeit von Schichtsilicaten so zu verbessern, daß ein pulverförmiges Material auch bei niedrigen bis mittleren Scherkräften ohne weiteres stippenfrei dispergiert werden kann. Weiterhin sollen pumpbare Pasten, die leicht dosierbar sind und in denen das Schichtsilicat vollkommen dispergiert vorliegt, bereitgestellt werden.

Gegenstand der Erfindung ist ein Verdickungsmittel auf der Basis mindestens eines synthetischen Schichtsilicats, das dadurch gekennzeichnet ist, daß es mindestens einen Zusatz aus der Gruppe der nachstehend angegebenen organischen Phosphorverbindungen
a) Phosphonsäuren mit der Formel 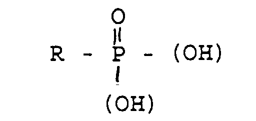
b) Phosphinsäuren mit der Formel 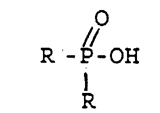
c) Thiophosphinsäuren mit der Formel 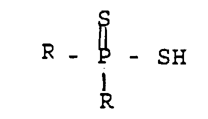
d) Diester der phosphorigen Säure mit der Formel

   HO - P (OR)₂
e) Diester der Phosphorsäure mit der Formel 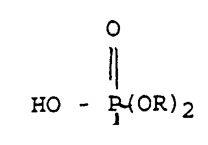
f) Diphosphonsäuren mit der Formel 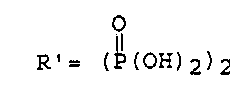
worin R eine Alkyl- oder Hydroxyalkylgruppe mit 1 bis 22 C-Atomen oder eine Aryl- oder Hydroxyarylgruppe mit 6 bis 12 C-Atomen und R'eine Alkylengruppe mit 2 bis 22 C-Atomen darstellt, enthält.

Überraschenderweise wurde gefunden, daß durch die Verwendung der erfindungsgemäßen Verdickungsmittel die gestellte Aufgabe dadurch gelöst werden kann, daß diese neuartigen Produkte entweder als Pulver direkt in die fertige Formulierung stippenfrei eindispergiert werden können, oder daß die Viskosität dieser Verdickungsmittel in Wasser so reduziert wird, daß auch pumpbare und dennoch hochkonzentrierte Pasten herstellbar sind, ohne daß die Effizienz des Verdickungsmittels im System geschmälert wird.

Durch die erfindungsgemäßen Verdickungsmittel ist es auch möglich, Pulver in fertige Systeme nachträglich einzuarbeiten, ohne extreme Scherkräfte anwenden zu müssen.

Gegenstand der Erfindung ist ferner ein Verfahren zur Herstellung des erfindungsgemäßen Verdickungsmittels, wobei der Zusatz entweder
(a) als Pulver zum trockenen Pulver des Schichtsilicats gegeben wird; oder
(b) als Pulver oder in gelöster Form als Flüssigkeit auf einer geeigneten Stufe bei der Synthese des Schichtsilicats, vorzugsweie dem Filterkuchen, zugesetzt wird.

Gegenstand der Erfindung ist ferner eine Stammpaste, die aus dem erfindungsgemäßen Verdickungsmittel hergestellt wird und die bis zu etwa 35 Gew.-%, vorzugsweise etwa 5 bis 25 Gew.-%, insbesondere etwa 10 bis 20 Gew.-% Feststoff enthält.

Schließlich ist Gegenstand der Erfindung die Verwendung des erfindungsgemäßen Verdickungsmittels bzw. der Stammpaste in flüssigen oder fließfähigen, d.h. durch Streichen, Spritzen, Rollen usw. auftragbaren Beschichtungssystemen, wie z.B. Farben und Lacken, kosmetischen und pharmazeutischen Präparaten (wie Cremes und Zahnpasten), Dügenmittelsuspensionen, Pestizidsprühsystemen, Unterbodenschutzmassen Bitumen-emulsionen, Schmierfetten, Poliermitteln, Druckfarben, Strukturputzen usw.. Damit wird die Rheologie eingestellt; insbesondere wird die Viskosität, die Stabilität (Antiabsetzwirkung, Synerese) und die Schichtdicke (Antiablaufwirkung) kontrolliert.

Bevorzugte Ausführungsformen der Erfindung sind in den Unteransprüchen angegeben.

Die Erfindung ist durch die nachstehenden Beispiele näher erläutert.

### Beispiel 1

3 Gew.-% eines synthetischen Hectorits werden in 97 Gew.-% entmineralisiertem Wasser dispergiert. Im Vergleich dazu werden 3 Gew.-% synthetischer Hectorit in entmineralisiertem Wasser mit 1 Gew.-% eines Zusatzmittels, bezogen auf die Hectorit-Zugabemenge (=0,03 % der Gesamtmenge) dispergiert. Gemessen wird die Viskosität in der Dispersion in mPa.s (Viscolab LC 10, Fa. Physica).

Die Ergebnisse sind in Tabelle I angegeben.

**Tabelle I**

| Verdickungsmittel | Einsatzmenge | Zusatzmittel | Einsatzmenge | Viskosit. | |
|---|---|---|---|---|---|
| | | | | 1 h | 24 h |
| synthet.Hectorit | 3% | 0 | 0 | 18500 | 26000 |
| " | 3% | Na-Polyphosphat | 1% | 16300 | 32000 |
| " | 3% | Na-Polyacrylat | 1% | 12000 | 20000 |
| " | 3% | Hydroxyethan-1,1-di-phosphonsäure | 1% | 4200 | 9200 |

### Beispiel 2

Analog Beispiel 1 wird ein 3%iges Gel aus synthetischem Hektorit in entmineralisiertem Wasser hergetellt; im Vergleich dazu werden Gele mit 3 Gew.-% Zusatzmittel (0,09 % der Gesamtmenge) hergestellt.

Die Viskosität der Gele ist in Tabelle II angegeben.

**Tabelle II**

| Verdickungsmittel | Einsatzmenge | Zusatzmittel | Einsatzmenge | Viskosit. | |
|---|---|---|---|---|---|
| | | | | 1 h | 24 h |
| synthet.Hectorit | 3% | 0 | 0 | 11700 | 19000 |
| " | 3% | Na-Polyphosphat | 3% | 9000 | 15000 |
| " | 3% | Na-Polyacrylat | 3% | 5800 | 10600 |
| " | 3% | Hydroxyethan-1,1-di-phosphonsäure | 3% | 4 | 4 |
| " | 3% | Na-Phosphonat | 3% | 2760 | 6800 |

Die Ergebnisse der Beispiele 1 und 2 zeigen, daß die erfindungsgemäßen organischen Phosphorverbindungen die Viskosität der Dispersionen wesentlich stärker herabsetzen als die üblichen Dispergierhilsfmittel auf Basis Na-Polyphosphat oder Na-Acrylat. Erkennbar ist auch, daß durch Erhöhung der Zugabemenge von 1% auf 3% des Zusatzmittels die verflüssigende Wirkung noch verstärkt wird.

### Beispiel 3

Synthetischer Hectorit von Beispiel 1 wird in entmineralisiertem Wasser dispergiert (Dissolver, 5 min, 2800 UpM, Fa. Pendraulik) und die Viskosität des erhaltenen Gels wird nach 1 h bzw. 24 h im Brookfield DV-II in mPa.s gemessen.

Im Vergleich dazu wird der synthetische Hektorit mit dem Na-Salz der Hydroxyethan-1,1-diphosphonsäure gemischt, homogenisiert und in entmineralisiertem Wasser dispergiert.

Die Ergebnisse sind in Tabelle III angegeben.

**Tabelle III**

| | Anteil synth. Hektorit | Anteil Zusatzmittel | Einsatzmenge in Wasser | Viskosität | |
|---|---|---|---|---|---|
| | | | | 1h | 24 h |
| a) | 100 % | 0% | 4% | 10600 | 19800 |
| b) | 100 % | 0% | 6% | 56500 | 81700 |
| c) | 96 % | 4% | 8% | 6 | 8 |
| d) | 94 % | 6% | 25% | 1260 | 96 |

### Beispiel 4

Analog Beispiel 3 wird der synthetische Hectorit mit Phosphonsäure (Sapetin D100 der Firma Woellner) bzw. mit Na-Polyphosphat versetzt, homogenisiert und in entmineralisiertem Wasser dispergiert. Die Viskosität wird gemessen im Viscolab LC 10 der Fa. Physica.

Die Ergebnisse sind in Tabelle IV angegeben.

**Tabelle IV**

| | Anteil synth. Hektorit | Anteil Zusatzmittel | Einsatzmenge in Wasser | Viskosität | |
|---|---|---|---|---|---|
| | | | | 1h | 24 h |
| a) | 100 % | 0% | 4% | 10600 | 19800 |
| b) | 100 % | 0% | 6% | 56500 | 81700 |
| c) | 94 % | 6% Phosphonsr. | 6% | 24600 | 42500 |
| d) | 94 % | 6% Na-Polyphosphat | 6% | 16800 | 28300 |
| e) | 94 % | 6% Phosphonsäure | 4% | 6600 | 12600 |
| f) | 94 % | 6% Na-Polyphosphat | 4% | 2060 | 7080 |

### Beispiel 5

Analog Beispiel 3 und 4 wird der synthetische Hektorit mit den Zusatzmitteln homogen vermischt und mit dem Mischprodukt ein Gel in Leitungswasser (im Dissolver 5 min bei 2800 UpM gerührt) hergestellt und die Viskosität gemessen (Vicolab LC 10).

Die Ergebnisse sind in Tabelle V angegeben.

**Tabelle V**

| | Anteil synth. Hektorit | Anteil Zusatzmittel | Einsatzmenge in Wasser | Viskosität | |
|---|---|---|---|---|---|
| | | | | 1h | 24 h |
| a) | 100 % | 0% | 4% | 35800 | 77600 |
| b) | 100 % | 0% | 6% | 85300 | 163700 |
| c) | 94 % | 6% Phosphonsr. | 4% | 5420 | 7800 |
| d) | 94 % | 6% Na-Polyphosphat | 6% | 17100 | 25900 |

Die Beispiele 4 und 5 zeigen deutlich, daß zwar auch durch Zugabe von Standard-Dispergierhilfsmitteln die Viskosität - vor allem im Leitungswasser - reduzierbar ist. Die verflüssigende Wirkung des Zusatzmittels nach Beispiel 3 wird jedoch bei weitem nicht erreicht.

### Beispiel 6

Da durch die Zusatzmittel die Viskosität der Stammpasten, d.h. der in Wasser dispergierten Verdickungsmittel, stark abgesenkt wird, bleibt nachzuweisen, daß das Verdickungsmittel auch im fertigen System, wie z.B. einem Lack, tatsächlich seine Wirksamkeit entfaltet.

Dies wurde mit nachfolgender Dekorfarbe (Richtrezeptur C-823 der Fa. ICI) getestet:

| | | | |
|---|---|---|---|
| Rezeptur: | - Propylenglykol | 5,50 | Gew.-Teile |
| | - Wasser | 7,75 | " " |
| | - AMP-95 ® | 0,50 | |
| | - Dehydran 1293 ® | 1,25 | |
| | - Surfynol 104 E ® | 1,00 | |
| | - Neocryl BT-24 ® | 7,25 | |
| | - TiO₂ (Tioxide TR-92) | 56,25 | |
| Dispergieren auf < 10 µm | | | |
| | - Wasser | 8,50 | |
| | - Neocryl AH-24-C ® | 25,00 | 3 min, 930 UpM |
| | - Rheol. Zus. s. Tab. VI | x | 10 min, 3720 UpM |
| | - Neocryl AH-24-C ® | 113,75 | 5 min, 930-2800 UpM |
| | - Diethylenglykol ® | 15,50 | 5 min, 930-1865 UpM |
| | - Dehydron 1293 ® | 2,25 | |
| | - Byk 154 ® | 1,75 | 5 min, 930-1865 UpM |

**Tabelle VI**

| | synth. Hectorit | Anteil Zusatzmittel | Zugabemenge in Rezeptur | Viskosität mPa.s | Korn µm | Glanz 60°< | Ablauf bei µm |
|---|---|---|---|---|---|---|---|
| a) | 100% | - | 0,9% | 144 | 100 | 54,3 | 150 |
| b) | 95% | 5% Na-Polyphosphat | 0,9% | 188 | 50 | 50,5 | 150 |
| c) | 95% | 5% Phosphonsäure | 0,9% | 415 | 100 | 52,2 | 175 |
| d) | 95% | 5% Hydroxyethan-1,1-diphosphonsäure | 0,9% | 2841 | 40 | 62,2 | 300 |

Die Einarbeitung erfolgte als Pulver. Vor allem Beispiel (d) zeigt die ausgezeichnete Wirkung des Verdickungsmittels im System.

### Beispiel 7

Die Wirkung der homogenisierten Mischprodukte wurde auch in einem handelsüblichen, wasserverdünnbaren Acryl-Heizkörperlack überprüft. In diesen gekauften Lack (Handelsprodukt der Firma Südwest Lacke & Farben) wurde das Verdickungsmittel als Pulver (im Dissolver, 15 min. Rühren, 2800 UpM) eingearbeitet.

Heizkörperlack unverdünnt: 1700 mPa.s Glanz (60°<): 79,5

| Synth. Hektorit | Anteil Zusatzmittel | Zugabemenge zum Lack | Viskosität | Glanz 60°< |
|---|---|---|---|---|
| 100% | - | 2% | 58.200 | 42,5 |
| 94% | 6% Hydroxyethandiphosphonsäure-Na-Salz | 2% | 98.400 | 57,8 |
| 94% | 6% Na-Polyphosphat | 2% | 55.500 | 49,1 |
| 96% | 4% Hydroxyethandiphosphonsäure-Na-Salz | 2% | 89.600 | 55,2 |
| 98% | 2% Hydroxyethandiphosphonsäure-Na-Salz | 2% | 85.200 | 47,0 |

### Beispiel 8

Die erfindungsgemäßen rheologischen Additive wurden auch in einer Holzschutzlasur in folgender Formulierung getestet:

| | Gew.-Teile: |
|---|---|
| - Wasser | 9,4 |
| - rheolog. Zus.s.Tab.VIII | 20,0 - als 4%ige Stammpaste in Wasser |
| - Lumiten i. RBD ® | 0,4 - 10 min, Dissolver, 930 UpM |
| - Pigmentverteiler A ® | 0,4 |
| - Acronal A 627 ® | 136,0 |
| - Agitan 702 ® | 0,8 |
| - Propylenglykol | 10,0 |
| - Solvenon PP ® | 4,0 - 10 min, 930 UpM |
| - Mergal S 96 ® | 6,0 |
| - Luconyl-Wasserteig ® | 10,0 - 5 min, 930 UpM |

**Tabelle VIII**

| synth. Hectorit | Anteil Zusatzmittel | Einsatzmenge in Rezeptur | Viskosität mPa.s | Korn µm | Glanz 60°< |
|---|---|---|---|---|---|
| 100 % | - | 0,4% | 153 | 100 | 38,8 |
| 94 % | 6% Na-Polyphosphat | 0,4% | 175 | 90 | 60,6 |
| 94 % | 6% Hydroxyethan-1,1-diphosphonsäure | 0,4% | 148 | 15 | 75,7 |

Die Beispiele 6 bis 8 zeigen eindeutig, daß die rheologischen Verdickungsmittel, die aus einem synthetischen Schichtsilicat durch Zugabe eines Zusatzmittels modifiziert wurden, auch in Beschichtungssystemen eine ausgezeichnete Wirkung aufweisen. Trotz der zum Teil sehr stark ausgeprägten "Verflüssigung" (Viskositätserniedrigung) der Stammpaste bleibt im System die Wirkung erhalten bzw. es wird die Wirkung im Vergleich zur unmodifizierten Variante sogar besser.

## Patentansprüche

1. Verdickungsmittel auf der Basis mindestens eines synthetischen Schichtsilicats, dadurch gekennzeichnet, daß es mindestens einen Zusatz aus der Gruppe der nachstehend angegebenen organischen Phosphorverbindungen
a) Phosphonsäuren mit der Formel 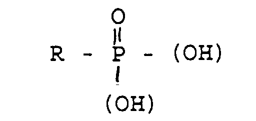
b) Phosphinsäuren mit der Formel 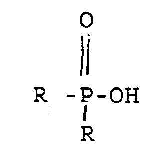
c) Thiophosphinsäuren mit der Formel 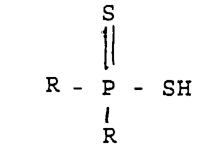
d) Diester der phosphorigen Säure mit der Formel
HO - P (OR)₂
e) Diester der Phosphorsäure mit der Formel 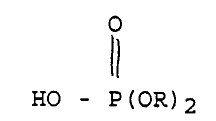
f) Diphosphonsäuren mit der Formel 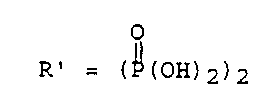
worin R eine Alkyl- oder Hydroxyalkylgruppe mit 1 bis 22 C-Atomen oder eine Aryl- oder Hydroxyarylgruppe mit 6 bis 12 C-Atomen und R' eine Alkylengruppe mit 2 bis 22 C-Atomen darstellt, enthält.

2. Verdickungsmittel nach Anspruch 1, dadurch gekennzeichnet, daß der Anteil des Zusatzes weniger als etwa 20 Gew.-% beträgt.

3. Verdickungsmittel nach Anspruch 1 oder 2, dadurch gekennzeichnet, daß der Anteil des Zusatzes zwischen etwa 1 Gew.-% und 15 Gew.-%, vorzugsweise zwischen 2 und 10 Gew.-%, insbesondere zwischen 4 und 8 Gew.-% liegt.

4. Verdickungsmittel nach einem der Ansprüche 1 bis 3, dadurch gekennzeichnet, daß der Zusatz als Alkalisalz, insbesondere als Na-Salz, vorliegt.

5. Verfahren zur Herstellung eines Verdickungsmittels nach einem der Ansprüche 1 bis 4, dadurch gekennzeichnet, daß der Zusatz als Pulver zum trockenen Pulver des Schichtsilicats zugegeben wird.

6. Verfahren zur Herstellung eines Verdickungsmittels nach einem der Ansprüche 1 bis 4, dadurch gekennzeichnet, daß der Zusatz als Pulver oder in gelöster Form als Flüssigkeit auf einer geeigneten Stufe bei der Synthese des Schichtsilicats, vorzugsweise dem Filterkuchen, zugesetzt wird.

7. Stammpaste, hergestellt aus dem Verdickungsmittel nach einem der Ansprüche 1 bis 4 und Wasser, dadurch gekennzeichnet, daß die Paste bis zu etwa 35 Gew.-%, vorzugsweise etwa 5 bis 25 Gew.-%, insbesondere etwa 10 bis 20 Gew.-% Feststoff enthält.

8. Stammpaste nach Anspruch 7, dadurch gekennzeichnet, daß sie den Zusatz in einer Menge von weniger als etwa 20 Gew.-%, bezogen auf das Schichtsilicat, enthält.

9. Verwendung des Verdickungsmittels nach einem der Ansprüche 1 bis 6 bzw. der Stammpaste nach Anspruch 7 oder 8, in flüssigen oder fließfähigen, d.h. durch Streichen, Spritzen, Rollen, usw. auftragbaren Beschichtungssystemen.

## Claims

1. A thickening agent based on at least one synthetic layered silicate, characterised in that it contains at least one additive from the group of the organic phosphorus compounds indicated below
a) phosphonic acids of the formula 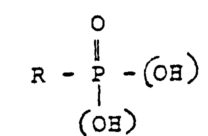
b) phosphinic acids of the formula 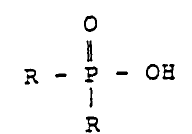
c) thiophosphinic acids of the formula 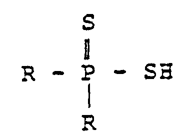
d) diesters of phosphorous acid of the formula
HO - P (OR)₂
e) diesters of phosphoric acid of the formula 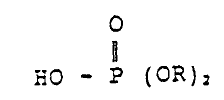
f) diphosphonic acids of the formula 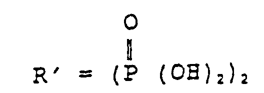
wherein R represents an alkyl or hydroxyalkyl group with 1 to 22 C atoms or an aryl or hydroxyaryl group with 6 to 12 C atoms and R' represents an alkylene group with 2 to 22 C atoms.

2. A thickening agent according to Claim 1, characterised in that the proportion of the additive is less than about 20 % by weight.

3. A thickening agent according to Claim 1 or 2, characterised in that the proportion of the additive is between approximately 1 % by weight and 15 % by weight, preferably between 2 and 10 % by weight, in particular between 4 and 8 % by weight.

4. A thickening agent according to any one of Claims 1 to 3, characterised in that the additive is present as an alkali salt, in particular as Na salt.

5. A method of preparing a thickening agent according to any one of Claims 1 to 4, characterised in that the additive is added as a powder to the dry powder of the layered silicate.

6. A method of preparing a thickening agent according to any one of Claims 1 to 4, characterised in that the additive is added as a powder or in dissolved form as a liquid at a suitable stage in the synthesis of the layered silicate, preferably to the filter cake.

7. A master paste, prepared from a thickening agent according to any one of Claims 1 to 4 and water, characterised in that the paste contains up to approximately 35 % by weight, preferably approximately 5 to 25 % by weight, in particular approximately 10 to 20 % by weight, of solid.

8. A master paste according to Claim 7, characterised in that it contains the additive in a quantity of less than approximately 20 % by weight, in relation to the layered silicate.

9. Use of a thickening agent according to any one of Claims 1 to 6 and/or of a master paste according to Claim 7 or 8, in liquid or flowable coating systems which can be applied by brushing, spraying, rolling, etc.

## Revendications

1. Epaississant à base d'au moins un phyllosilicate synthétique, caractérisé en ce qu'il contient au moins un additif choisi dans le groupe constitué par les composés organiques du phosphore indiqués ci-après :
a) acides phosphoniques de formule 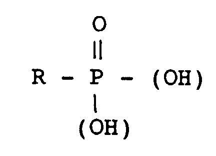
b) acides phosphiniques de formule 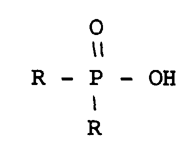
c) acides thiophosphiniques de formule 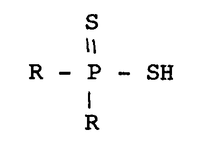
d) diesters de l'acide phosphoreux de formule
HO - P (OR)₂
e) diesters de l'acide phosphorique de formule 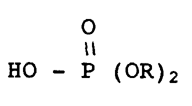
f) acides diphosphoniques de formule 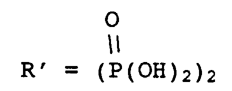
où R est un groupe alkyle ou hydroxyalkyle ayant de 1 à 22 atomes de carbone ou un groupe aryle ou hydroxyaryle ayant de 6 à 12 atomes de carbone, et R' est un groupe alkylène ayant de 2 à 22 atomes de carbone.

2. Epaississant selon la revendication 1, caractérisé en ce que la proportion de l'additif est inférieur à environ 20 % en poids.

3. Epaississant selon la revendication 1 ou 2, caractérisé en ce que la proportion de l'additif est comprise entre 1 et 15 % en poids, de préférence entre 2 et 10 % en poids, en particulier entre 4 et 8 % en poids.

4. Epaississant selon l'une des revendications 1 à 3, caractérisé en ce que l'additif se présente sous forme d'un sel d'un métal alcalin, en particulier d'un sel de Na.

5. Procédé de préparation d'un épaississant selon l'une des revendications 1 à 4, caractérisé en ce que l'additif est ajouté sous forme d'une poudre à la poudre sèche du phyllosilicate.

6. Procédé de préparation d'un épaississant selon l'une des revendications 1 à 4, caractérisé en ce que l'additif est ajouté sous forme d'une poudre ou sous forme dissoute, sous forme d'un liquide, à un stade approprié lors de la synthèse du phyllosilicate, de préférence sur le gâteau de filtration.

7. Pâte mère, préparée à partir d'un épaississant selon l'une des revendications 1 à 4 et d'eau, caractérisée en ce que la pâte présente une teneur en extrait sec allant jusqu'à environ 35 % en poids, de préférence d'environ 5 à 25 % en poids, en particulier d'environ 10 à 20 % en poids.

8. Pâte mère selon la revendication 7, caractérisée en ce qu'elle contient l'additif en une quantité inférieure à environ 20 % en poids par rapport au phyllosilicate.

9. Utilisation de l'épaississant selon l'une des revendications 1 à 6 ou de la pâte mère selon la revendication 7 ou 8 dans des systèmes de revêtement liquides ou fluides, c'est-à-dire pouvant être appliqués par induction, au pistolet, au rouleau, etc.
